# EUROPÄISCHE PATENTSCHRIFT

(11) **EP 3 316 817 B1**
(45) Veröffentlichungstag und Bekanntmachung des Hinweises auf die Patenterteilung: **29.12.2021**
(21) Anmeldenummer: 16736023.9
(22) Anmeldetag: 29.06.2016
(51) Int. Cl.: A61C 17/02

(54) **BETRIEBSWASSERVERSORGUNGSEINHEIT ZUM VERSORGEN EINER MEDIZINISCHEN BEHANDLUNGSEINHEIT, ZAHNMEDIZINISCHE BEHANDLUNGSEINHEIT UND BETRIEBSVERFAHREN**
PROCESS WATER SUPPLY UNIT FOR SUPPLYING A MEDICAL TREATMENT UNIT, DENTAL MEDICAL TREATMENT UNIT AND OPERATING METHOD
UNITÉ D'ALIMENTATION EN EAU INDUSTRIELLE POUR ALIMENTER UNE UNITE DE TRAITEMENT MÉDICAL, UNITÉ DE TRAITEMENT DENTAIRE ET PROCÉDÉ DE FONCTIONNEMENT

(30) Priorität: 30.06.2015 DE 102015212248
(43) Veröffentlichungstag der Anmeldung: 09.05.2018
(73) Patentinhaber: BLUE SAFETY GmbH, 48153 Münster (DE)
(72) Erfinder: PAPENBROCK, Jan, 48167 Münster (DE); FISCHER, Sebastian, 48145 Münster (DE); MÖNNINGHOFF, Christian, 48151 Münster (DE); MAASS, Mathias, 59077 Hamm (DE)
(74) Vertreter: Gulde & Partner
(86) Internationale Anmeldenummer: PCT/EP2016/065157
(87) Internationale Veröffentlichungsnummer: WO 2017/001486

(56) Entgegenhaltungen:
- EP-A1- 0 395 557
- EP-A2- 1 468 659
- WO-A1-2009/016655
- JP-A- H11 104 149
- US-A- 5 526 841
- US-A1- 2003 036 033

## Beschreibung

Die vorliegende Erfindung betrifft eine Betriebswasserversorgungseinheit und ein Betriebsverfahren zur Versorgung einer medizinischen Behandlungseinheit mit einem bevorzugt biozidhaltigem Betriebswasser. Die vorliegende Erfindung betrifft ferner eine zahnmedizinische Behandlungseinheit. Die zahnmedizinische Behandlungseinheit kann ein rotierendes Instrument in Form eines Bohrers sein oder einen solchen aufweisen. Das Betriebswasser wird typischerweise zum Kühlen des Instruments und zum Spülen einer Patienten Mundhöhle verwendet.

Betriebswasserversorgungseinheiten des Standes der Technik weisen eine Behälteraufnahme zum Aufnehmen eines Druckwasserbehälters und einen Drucklufteingang zum Anschließen der Betriebswasserversorgungseinheit an eine externe Druckluftquelle, beispielsweise einen Druckluftkompressor in einer zahnmedizinischen Praxis, auf. Ferner ist ein Druckluftanschluss vorgesehen über den von dem Drucklufteingang stammende Druckluft in den Druckwasserbehälter eingeleitet werden kann. Bekannte Betriebswasserversorgungseinheiten verfügen des weiteren über einen Betriebswasseranschluss über den das Betriebswasser, das durch die in den Druckwasserbehälter eingeleitete Druckluft aus dem Druckwasserbehälter herauszufördern ist, an die zahnmedizinische Behandlungseinheit abgegeben werden kann. Bekannt sind, beispielsweise aus der EP 1 468 659 A2, auch Zahnpflegegeräte für den Heimgebrauch zum Strahlen der Zähne.

WO 2009/016655 A1 beschreibt ein Inhalationsgerät mit einem Inhalatorkopf zur Aufnahme von Wasser, der durch einen Kompressor mit Druckluft beaufschlagt wird. Ein Luftfilter kann innerhalb der Luftleitung, im Kompressorausgang oder am Eingang des Inhalatorkopfs angeordnet sein.

EP 0 395 557 A1 beschreibt ein Zahnpflegegerät mit einem Handstück, dem Wasser und/oder ein Abrasionsmittel zugeführt werden. Dies erfolgt jeweils mit Druckluft, die zuvor durch ein Trocknungsfilter getrocknet wird.

Aus US 2003/0036033 A1 ist eine zahnmedizinische Betriebswasserversorgungseinheit bekannt, bei der Wasser und Druckluft jeweils durch einen vorgeschalteten Filter aufbereitet werden.

US 5,526,841 A offenbart eine zahnmedizinische Betriebswasserversorgungseinheit mit einem kastenförmigen Gehäuse, in welchem verschiedene Leitungen für Druckluft und Flüssigkeiten sowie ein Luftfilter angeordnet sind. Es sind zwei an dem Gehäuse angeordnete Anschlüsse für einen Wasserbehälter und einen Desinfektionsmittelbehälter vorgesehen, die wahlweise mit der Druckluft zur Förderung des jeweiligen Inhalts an eine zahnmedizinische Behandlungseinheit beaufschlagt werden können.

Es ist Aufgabe der vorliegenden Erfindung eine Betriebswasserversorgungseinheit, eine zahnmedizinische Behandlungseinheit sowie ein Betriebsverfahren anzugeben, die besonders hygienisch, sicher und kostengünstig sind.

Die erfindungsgemäße Betriebswasserversorgungseinheit zum Versorgen einer medizinischen Behandlungseinheit mit Betriebswasser, umfasst: einen Druckwasserbehälter zur Bevorratung von Betriebswasser; eine Behälteraufnahme, die zur Aufnahme des Druckwasserbehälters ausgebildet ist; und ein zylinderförmiges Deckelteil, das die Behälteraufnahme einhaust. Die Behälteraufnahme umfasst: einen Drucklufteingang, der zum Anschluss der Betriebswasserversorgungseinheit an eine Druckluftquelle ausgebildet ist; einen Druckluftanschluss, der ausgebildet ist, die von dem Drucklufteingang stammende Druckluft in den Druckwasserbehälter einzuleiten; einen Betriebswasseranschluss, der ausgebildet ist, das Betriebswasser durch die in den Druckwasserbehälter eingeleitete Druckluft aus dem Druckwasserbehälter herauszufördern und an die medizinische Behandlungseinheit abzugeben; einen Sterilluftfilter, der in einen Druckluftpfad zwischen dem Drucklufteingang und dem Druckluftanschluss eingebunden ist; und eine tellerförmige, runde Grundplatte, durch die der Betriebswasseranschluss und der Druckluftanschluss zumindest abschnittsweise hindurch verlaufen.

Die Erfindung schließt die Erkenntnis ein, dass eine Drucklufterzeugung in Zahnarztpraxen typischerweise durch zentrale Druckluftkompressoren erfolgt. Damit besteht die Gefahr, dass dort befindliche Schmutzpartikel und Mikroorganismen angesaugt und über ein Druckluftleitungssystem einer Behandlungseinheit und somit dem Patienten zugeführt werden. Zur Vermeidung dieses Nachteils wurden im Stand der Technik Kompressoren mit integrierten Luftfiltern vorgeschlagen.

In Abkehr dazu weist die erfindungsgemäße Betriebswasserversorgungseinheit einen Sterilluftfilter auf. Somit kann vorteilhafterweise ein Infektionsschutz am unmittelbaren Behandlungsort bereitgestellt werden. Etwaige Schäden am Druckluftleitungssystem und/oder am Kompressor oder eine unsachgemäße Wartung selbiger kann wirksam kompensiert werden. Somit ist die erfindungsgemäße Betriebswasserversorgungseinheit besonders sicher.

In einer bevorzugten Ausgestaltung weist der Sterilluftfilter eine Porengröße von < 90 µm (Mikrometer) auf. Es hat sich als vorteilhaft herausgestellt, wenn die Porengröße < 50 µm, besonders bevorzugt < 10 µm beträgt. In einer besonders bevorzugten Ausgestaltung weist der Sterilluftfilter eine Porengröße von < 0,2 µm auf.

Um eine besonders kompakte Betriebswasserversorgungseinheit bereitzustellen, hat es sich als vorteilhaft herausgestellt, wenn der Sterilluftfilter erfindungsgemäß innerhalt eines Gehäusevolumens der Betriebswasserversorgungseinheit angeordnet ist. Erfindungsgemäß ist der Sterilluftfilter in den Druckluftpfad zwischen dem Drucklufteingang und dem Druckluftanschluss eingebunden.

Bevorzugt erfolgt eine Anordnung des Sterilluftfilters endständig bezogen auf einen Druckluftschlauch, der die externe Druckluftquelle mit dem Druckluftanschluss der Betriebswasserversorgungseinheit verbindet.

Bevorzugt ist ein Druckluftpfad zwischen der externen Druckluftquelle, d. h. beispielsweise einem Kompressor, und dem Sterilluftfilter ohne zusätzliche Filterelemente ausgestattet, d. h. filterfrei. Alternativ zu einer unmittelbar endständigen Anordnung des Sterilluftfilters im Druckluftpfad zwischen der externen Druckluftquelle und dem Druckluftanschluss kann der Sterilluftfilter in der Nähe des Druckluftanschlusses angeordnet sein.

Bevorzugt beträgt die Länge einer Druckluftleitung zwischen einem außerhalb der Betriebswasserversorgungseinheit angeordneten Sterilluftfilter und dem Druckluftanschluss am oder im Gehäuse der Betriebswasserversorgungeinheit < 5 m, bevorzugt < 2 m, weiter bevorzugt < 1 m.

In einer bevorzugten Ausgestaltung weist die Betriebswasserversorgungseinheit einen Schalter auf, mittels dem der Druckluftpfad zwischen dem Drucklufteingang und dem Druckluftanschluss unterbrochen und/oder freigeschaltet werden kann. Bevorzugt ist der Schalter dem Sterilluftfilter nachgeschaltet, vorzugsweise unmittelbar nachgeschaltet. Alternativ kann der Schalter dem Sterilluftfilter vorgeschaltet sein.

Die Betriebswasserversorgungseinheit weist ein zylinderförmiges Deckelteil auf, das den Sterilluftfilter einhaust, bevorzugt vollständig ein. Die Betriebswasserversorgungseinheit weist eine tellerförmige, runde Grundplatte auf. Die tellerförmige, runde Grundplatte bildet zumindest teilweise die Behälteraufnahme zum Aufnehmen des Druckwasserbehälters. Der Betriebswasseranschluss und der Druckluftanschluss verlaufen zumindest abschnittsweise durch die tellerförmige Grundplatte hindurch.

Es hat sich als vorteilhaft herausgestellt, wenn die tellerförmige Grundplatte eine umlaufende Dichtung aufweist oder der tellerförmigen Grundplatte eine umlaufende Dichtung zugeordnet ist. Die umlaufende Dichtung kann den Druckwasserbehälter zur Umgebung hin abdichten, wenn diese an der Behälteraufnahme befestigt ist. Die umlaufende Dichtung ist vorzugsweise ein Dichtring.

In einer besonders bevorzugten Ausgestaltung ist die tellerförmige Grundplatte vollständig innerhalb des zylinderförmigen Deckelteils angeordnet. Derart ergibt sich ein besonders kompakter und einfach zu reinigender Aufbau der Betriebswasserversorgungseinheit.

Das zylinderförmige Deckelteil kann an seinem inneren Umfang wenigstens ein Rastelement aufweisen, in das ein korrespondierendes Rastelement des Druckwasserbehälters zum Halten des Druckwasserbehälters an der Behälteraufnahme einrasten kann. Bevorzugt ist das Rastelement am inneren Umfang des zylinderförmigen Deckelteils als Rampe bzw. rampenförmig ausgebildet. Am inneren Umfang des zylinderförmigen Deckelteils können, bevorzugt gleichmäßig voneinander beabstandet, mehrere Rastelemente angeordnet sein. Bevorzugt sind am inneren Umfang des zylinderförmigen Deckelteils drei rampenförmige Rastelemente angeordnet.

Es hat sich als vorteilhaft herausgestellt, wenn der Druckwasserbehälter, der seinerseits bevorzugt zylinderförmig ausgebildet ist, an seinem äußeren Umfang wenigstens ein korrespondierendes Rastelement aufweist. Bevorzugt ist das korrespondierende Rastelement eine Rastnase, die ausgebildet ist, in ein rampenförmiges Rastelement am inneren Umfang des zylinderförmigen Deckelteils einzurasten. Auf Seiten des Druckwasserbehälters können mehrere, bevorzugt drei korrespondierende Rastelemente vorgesehen sein.

In einer weiteren bevorzugten Ausgestaltung weist die Betriebswasserversorgungseinheit einen Sterilwasserfilter auf. Der Sterilwasserfilter kann dem Betriebswasseranschluss vorgeschaltet oder nachgeschaltet sein, bevorzugt unmittelbar. In einer bevorzugten Ausgestaltung weist der Sterilwasserfilter eine Porengröße von < 90 µm (Mikrometer) auf. Es hat sich als vorteilhaft herausgestellt, wenn die Porengröße < 50 µm, besonders bevorzugt < 10 µm beträgt. In einer besonders bevorzugten Ausgestaltung weist der Sterilwasserfilter eine Porengröße von < 0,2 µm auf.

Besonders bevorzugt ist der Sterilwasserfilter bezüglich eines Betriebswasserpfades in der Betriebswasserversorgungseinheit endständig angeordnet. Mittels eines vorgesehenen Sterilwasserfilters kann ein in der Betriebswasserversorgungseinheit vorgesehenes biozidfreies Betriebswasser beim Austritt aus der Betriebswasserversorgungseinheit wirksam entkeimt werden. Selbstverständlich kann der Sterilwasserfilter auch für den Fall vorgesehen sein, dass biozidhaltiges Betriebswasser zum Einsatz kommt.

In einer besonders bevorzugten Ausgestaltung ist der Druckwasserbehälter zur Bevorratung des Betriebswassers von der Betriebswasserversorgungseinheit umfasst. Es hat sich als vorteilhaft herausgestellt, wenn der Druckwasserbehälter ein Volumen zwischen 100 ml und 5000 ml (Milliliter) aufweist. Das Volumen des Druckwasserbehälters kann zwischen 1000 ml und 2000 ml betragen, bevorzugt zwischen 1000 ml und 1300 ml betragen. Bevorzugt ist der Druckwasserbehälter zylinderförmig ausgebildet. Besonders bevorzugt ist Druckwasserbehälter druckfest bis wenigstens 3 Bar, bevorzugt 5 Bar. Bevorzugt ist der Druckwasserbehälter autoklavierbar. Der Druckwasserbehälter kann beispielsweise aus Kunststoff oder Metall bestehen.

Die Aufgabe wird ebenfalls gelöst durch eine zahnmedizinische Behandlungseinheit mit einer vorbeschriebenen Betriebswasserversorgungseinheit. Gleichsam wird die Aufgabe gelöst durch die Verwendung einer vorbeschriebenen Betriebswasserversorgungseinheit in einer zahnmedizinischen Behandlungseinheit.

Die Aufgabe wird ebenfalls gelöst durch eine HNO-Behandlungseinheit mit einer vorbeschriebenen Betriebswasserversorgungseinheit sowie durch die Verwendung einer Betriebswasserversorgungseinheit in der vorbeschriebenen Art in einer HNO-Behandlungseinheit.

In einer bevorzugten Ausgestaltung der zahnmedizinischen Behandlungseinheit (Dentaleinheit) ist der Betriebswasseranschluss der Betriebswasserversorgungseinheit mit einem Betriebswasserzulauf der zahnmedizinischen Behandlungseinheit verbunden. Über den Betriebswasserzulauf der zahnmedizinischen Behandlungseinheit kann das Betriebswasser einem Spülkopf oder einem Bohrer der zahnmedizinischen Behandlungseinheit zugeführt werden.

In einer weiteren bevorzugten Ausgestaltung ist der Drucklufteingang der Betriebswasserversorgungseinheit mit einem Druckluftausgang der zahnmedizinischen Behandlungseinheit verbunden. Die zahnmedizinische Behandlungseinheit kann selbst einen Kompressor oder ähnliches als Druckluftquelle aufweisen oder ihrerseits über einen Kompressor oder ein zentrales Druckluftsystem versorgt sein.

Bevorzugt ist auch bei der zahnmedizinischen Behandlungseinheit ein Sterilluftfilter innerhalb eines Gehäuses der umfassenden Betriebswasserversorgungseinheit angeordnet. Alternativ oder zusätzlich kann der Sterilluftfilter außerhalb des Gehäuses der Betriebswasserversorgungseinheit angeordnet sein und im Druckluftpfad, genauer gesagt im Druckluftschlauch, zwischen dem Druckluftausgang der zahnmedizinischen Behandlungseinheit und dem Drucklufteingang der Betriebswasserversorgungseinheit, angeordnet sein.

Die erfindungsgemäße HNO-Behandlungseinheit mit Betriebswasserversorgungseinheit kann entsprechend der zahnmedizinischen Behandlungseinheit weitergebildet sein.

Gemäß einem weiteren Aspekt der Erfindung wird die Aufgabe gelöst durch ein Verfahren zum Betreiben einer vorbeschriebenen Betriebswasserversorgungseinheit mit den Schritten:
- Führen einer insbesondere ungefilterten Druckluft von der Druckluftquelle zum Sterilluftfilter der Betriebswasserversorgungseinheit;
- Einleiten der durch den Sterilluftfilter der Betriebswasserversorgungseinheit gefilterten Druckluft in einen Druckwasserbehälter;
- wobei der Druckwasserbehälter während einer Behandlung mit einem bevorzugt biozidhaltigem Betriebswasser gefüllt ist, so dass das Betriebswasser über einen Betriebswasseranschluss der Betriebswasserversorgungseinheit abgegeben wird; und/oder
- wobei der Druckwasserbehälter während einer Reinigungsphase im Wesentlichen frei von Betriebswasser ist, so dass ein dem Sterilluftfilter nachgeschalteter Luftpfad mittels der vom Sterilluftfilter gefilterten Druckluft freigeblasen wird.

Durch das erfindungsgemäße Verfahren kann zum einen das Betriebswasser durch im Wesentlichen keimfreie Druckluft aus dem Druckwasserbehälter ausgetragen werden. Zum anderen kann eine der Betriebswasserversorgungseinheit nachgeschaltete zahnmedizinische Behandlungseinheit in einer Reinigungsphase mit steriler Druckluft freigeblasen werden. Dazu wird der Druckwasserbehälter entleert bzw. frei von Betriebswasser an der Betriebswasserversorgungseinheit befestigt und mit Druckluft beaufschlagt. Über den mit der zahnmedizinischen Behandlungseinheit verbundenen Betriebswasseranschluss, über den im Behandlungsbetrieb Betriebswasser geführt wird, gelangt nun sterile Druckluft in einen dem Betriebswasseranschluss nachgeschalteten Pfad. Vorteilhafterweise kann eine zahnmedizinische Behandlungseinheit oder einen HNO-Behandlungseinheit mit Sterilluft trockengeblasen werden. Dies ist beispielsweise über Nacht oder am Wochenende wünschenswert, um in diesen Zeiträumen eine Stagnation von Betriebswasser in den sonst wasserführenden Bauteilen oder Schläuchen der HNO-Behandlungseinheit oder der zahnmedizinischen Behandlungseinheit auszuschließen. Damit kann eine unerwünschte Besiedlung mit Biofilmen vermieden werden. Die sterile Luft, die mittels des Sterilluftfilters erzeugt wird, verhindert somit wirksam eine Kontamination der jeweiligen Behandlungseinheiten über Mikroorganismen und Nährstoffe, die über ungefilterte Druckluft typischerweise eingetragen wird.

Das erfindungsgemäße Verfahren kann durch Merkmale weitergebildet sein, die mit Bezug auf die Betriebswasserversorgungseinheit und/oder die medizinischen Behandlungseinheit beschrieben sind.

Ausführungsbeispiele der Erfindung werden nun nachfolgend anhand der Zeichnungen beschrieben. Weitere Vorteile, Merkmale und Einzelheiten der Erfindung ergeben sich aus der nachfolgenden Beschreibung der bevorzugten Ausführungsbeispiele sowie anhand der Zeichnungen; diese zeigen in:
- Fig. 1: eine schematische Darstellung eines Ausführungsbeispiels einer erfindungsgemäßen Betriebswasserversorgungseinheit;
- Fig. 2: eine schematische Darstellung einer Innenansicht der Betriebswasserversorgungseinheit aus Fig. 1;
- Fig. 3: eine Unteransicht der Darstellung aus Fig. 2;
- Fig. 4: eine schematische Darstellung eines zylinderförmigen Deckelteils der Betriebswasserversorgungseinheit aus Fig. 1;
- Fig. 5: das zylinderförmige Deckelteil der Fig. 4 mit eingesetzter Grundplatte;
- Fig. 6: eine perspektivische Darstellung eines Druckwasserbehälters der Betriebswasserversorgungseinheit der Fig. 1;
- Fig. 7: ein erstes Ausführungsbeispiel einer zahnmedizinischen Behandlungseinheit mit einer Betriebsversorgungseinheit;
- Fig. 8: ein zweites Ausführungsbeispiel einer zahnmedizinischen Behandlungseinheit mit einer Betriebswasserversorgungseinheit;
- Fig. 9: eine schematische Darstellung eines erfindungsgemäßen Verfahrens zum Betreiben einer Betriebswasserversorgungseinheit.

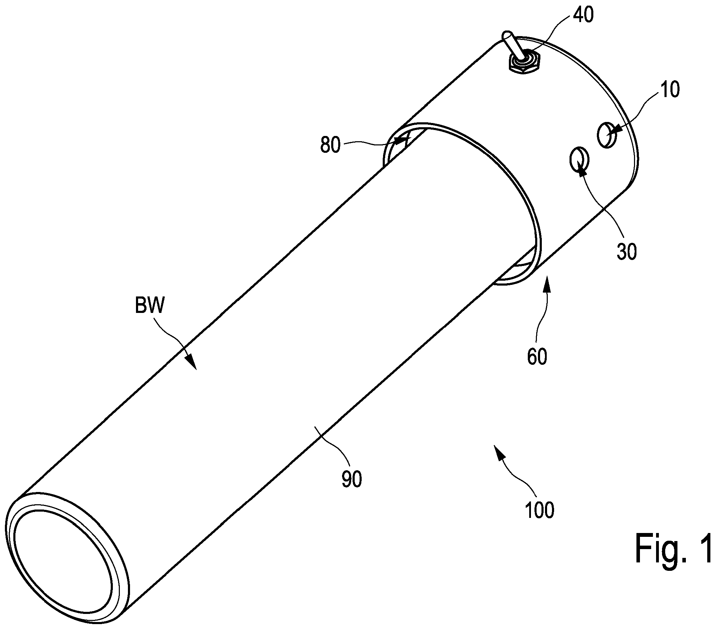

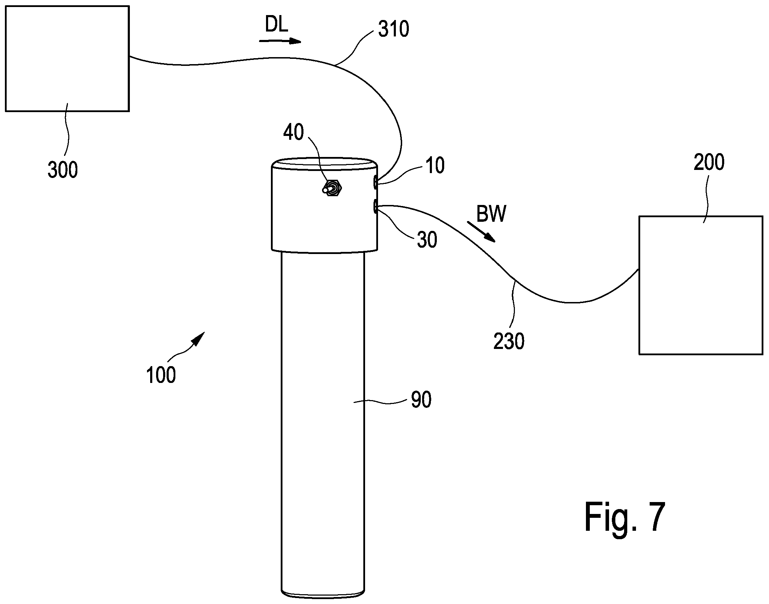

Eine Betriebswasserversorgungseinheit 100 ist in Fig. 1 gezeigt. Die Betriebswasserversorgungseinheit 100 weist ein zylinderförmiges Deckelteil 60 auf. Am Deckelteil 60 angeordnet ist ein Drucklufteingang 10 zum Anschließen der Betriebswasserversorgungseinheit 100 an eine Druckluftquelle 300 (vgl. Fig. 7). Ferner ist am zylinderförmigen Deckelteil 60 ein Betriebswasseranschluss 30 vorgesehen, über den Betriebswasser an eine medizinische Behandlungseinheit 200 (vgl. Fig. 7) abgegeben werden kann.

Die Betriebswasserversorgungseinheit 100 weist eine Behälteraufnahme 80 auf, an der im vorliegend dargestellten Ausführungsbeispiel ein zylinderförmig ausgebildeter Druckwasserbehälter 90 angeordnet ist. Im Druckwasserbehälter 90 ist das über den Betriebswasseranschluss 30 auszugebende biozidhaltige Betriebswasser BW eingefüllt. Über einen im zylinderförmigen Deckelteil 60 vorgesehenen Schalter 40 kann am Drucklufteingang 10 anliegende Druckluft freigeschaltet werden und das im Betriebswasserbehälter 90 vorgesehene Betriebswasser BW mittels Druckluft DL aus dem Betriebswasseranschluss 30 ausgeleitet werden.

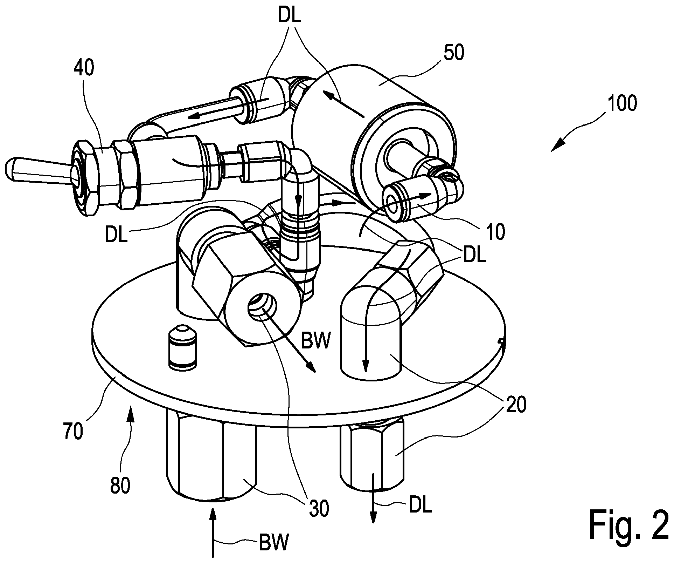

Fig. 2 zeigt die Innenansicht der Betriebswasserversorgungseinheit 100 der Fig. 1. Aus Gründen der Deutlichkeit wurde das zylinderförmige Deckelteil 60 (vgl. Fig.1) nicht dargestellt. Die Betriebswasserversorgungseinheit der Fig. 1 umfasst den Drucklufteingang 10, über den Druckluft DL in einen von der Betriebswasserversorgungseinheit 100 umfassten Sterilluftfilter 50 einströmen kann. Der Sterilluftfilter 50 ist im vorliegend dargestellten Ausführungsbeispiel innerhalb eines Gehäusevolumens, genauer gesagt innerhalb eines Volumens des zylinderförmigen Deckelteils 60 (vgl. Fig. 1), angeordnet.

Die Betriebswasserversorgungseinheit 100 weist weiter einen Druckluftanschluss 20 auf, über den von dem Drucklufteingang 10 stammende Druckluft DL strömen kann. Im vorliegend dargestellten Ausführungsbeispiel weist die Betriebswasserversorgungseinheit 100 eine tellerförmige Grundplatte 70 auf. Der Druckluftanschluss 20 verläuft zumindest abschnittsweise durch die Grundplatte hindurch. Ebenso weist die Betriebswasserversorgungseinheit 100 einen Betriebswasseranschluss 30 auf, der wie gezeigt abschnittsweise durch die tellerförmige Grundplatte 70 hindurch verläuft.

Eine Behälteraufnahme 80 zum Aufnehmen eines Druckwasserbehälters (vgl. Fig. 1) ist vorliegend durch die tellerförmige Grundplatte 70 gebildet. Das heißt, der unterseitig von der tellerförmigen Grundplatte 70 abragende Betriebswasseranschluss 30 sowie der unterseitig von der tellerförmigen Grundplatte 70 abragende Druckluftanschluss 20 ragen in ein Volumen des Druckwasserbehälters 90 hinein, wenn dieser an der Behälteraufnahme 80 angeordnet ist.

Wie der Fig. 2 entnommen werden kann, ist der Sterilluftfilter 50 dem Drucklufteingang 10 unmittelbar nachgestaltet. Dem Sterilluftfilter 50 wiederum unmittelbar nachgeschaltet ist ein Schalter 40, mittels dem der Druckluftpfad zwischen dem Drucklufteingang 10 und dem Druckluftanschluss 20 unterbrochen und freigegeben werden kann.

Im Folgenden soll die Funktion der erfindungsgemäßen Betriebswasserversorgungseinheit 100 näher erläutert werden. Eine von einer Druckluftquelle stammende Druckluft DL tritt in Pfeilrichtung in den Drucklufteingang 10 ein. Die in den Drucklufteingang 10 eintretende Druckluft DL ist im Wesentlichen ungefiltert, d. h. kann noch mit Keimen behaftet sein. Im weiteren Verlauf des Druckluftpfades gelangt die Druckluft DL durch den Sterilluftfilter 50, der vorliegend eine Porengröße von < 0,2 µm aufweist. Über eine Schlauchverbindung gelangt die Druckluft DL weiter zu dem Schalter 40, der im vorliegend dargestellten Ausführungsbeispiel in geöffneter Stellung gezeigt ist. Dementsprechend kann die Druckluft DL ungehindert weiter in Richtung des Druckluftanschlusses 20 strömen, um über diesen in den Druckwasserbehälter 90 (vgl. Fig. 1) zu gelangen.

Durch die in den Druckwasserbehälter 90 eingeleitete Druckluft DL wird Betriebswasser BW nach dem Verdrängungsprinzip in Pfeilrichtung in den Betriebswasseranschluss 30 auf der Unterseite der tellerförmigen Grundplatte 70 hineingedrückt, um auf dem oberhalb der tellerförmigen Grundplatte 70 befindlichen Teil des Betriebswasseranschlusses 30 aus diesem auszutreten. An dem oberseitig der tellerförmigen Grundplatte 70 befindlichen Teil des Betriebswasseranschlusses 30 wird typischerweise ein zu einer zahnmedizinischen Behandlungseinheit 200 führende Betriebswasserzuleitung 230 (vgl. Fig. 7) angeschlossen.

Es sei bemerkt, dass der Eingang des Drucklufteingangs 10 nicht, wie in Fig. 1 gezeigt, notwendigerweise am zylinderförmigen Deckelteil 60 angeordnet ist. Vielmehr kann das zylinderförmige Deckelteil 60 auch eine Durchführungsöffnung aufweisen, über die ein zum Anschluss an den Drucklufteingang 10 bestimmter Druckluftschlauch hindurchzuführen ist. Gleiches gilt für den Betriebswasseranschluss in entsprechender Weise.

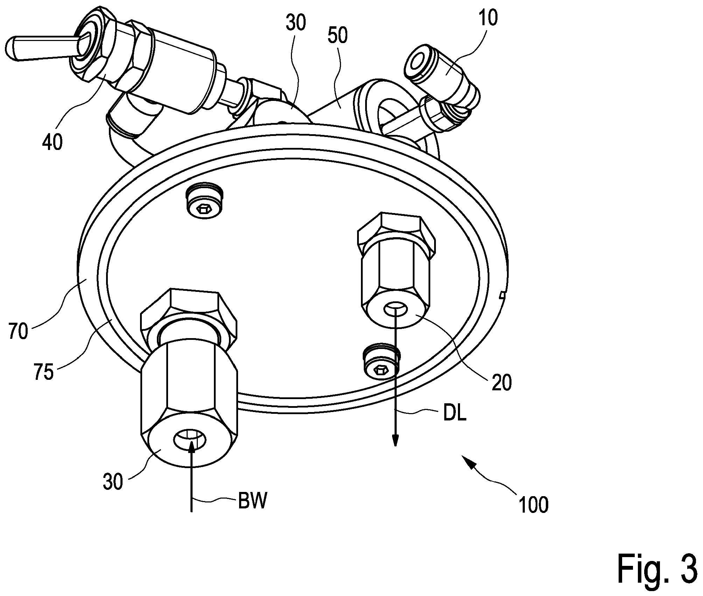

Fig. 3 zeigt die Betriebswasserversorgungseinheit 100 der Fig. 2 in Unteransicht, d.h. mit Blick aus Richtung des Druckwasserbehälters 90. Auf der Unterseite der tellerförmigen Grundplatte 70 gut zu erkennen sind die in den Druckwasserbehälter 90 (wenn montiert) hineinragenden Abschnitte des Druckluftanschlusses 20 bzw. des Betriebswasseranschlusses 30. Die Strömungsrichtung der Druckluft DL und die Strömungsrichtung des Betriebswassers BW sind in Pfeilrichtung gekennzeichnet.

Die tellerförmige Grundplatte 70 weist auf ihrer Unterseite eine umlaufende Dichtung 75, vorliegend einen Dichtring, auf. Durch die umlaufende Dichtung 75 wird ein Druckwasserbehälter 90, wenn dieser - wie in Fig. 1 gezeigt - im zylinderförmigen Deckelteil 60 angeordnet ist, zur Umgebung hin abgedichtet.

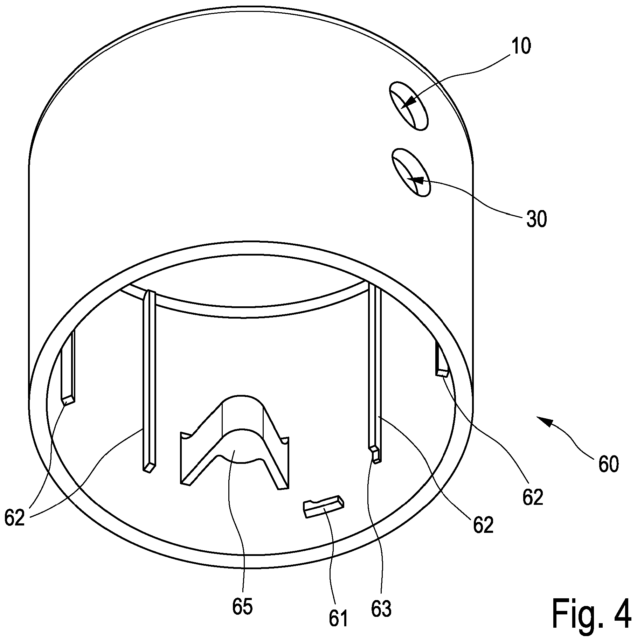

Fig. 4 zeigt nunmehr das zylinderförmige Deckelteil 60 in einer Unteransicht. Im oberen Teil des zylinderförmigen Deckelteils 60 sind Öffnungen für die Durchführung der zum Drucklufteingang 10 bzw. Betriebsanschluss 30 führenden Anschlussschläuche bezeichnet.

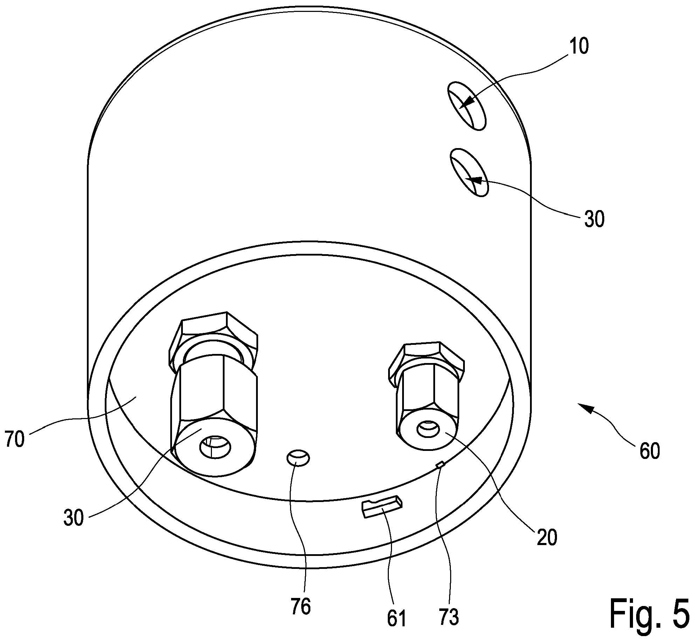

Am inneren Umfang des zylinderförmigen Deckelteils erstrecken sich axial zum zylinderförmigen Deckelteil 60 orientierte Stege 62, die einer Abstützung der tellerförmigen Grundplatte 70 (vgl. Fig. 5) dienen. Einer der Stege 62 weist eine unterseitige Ausnehmung 63 auf, die ein drehlagekorrektes Einsetzen der tellerförmigen Grundplatte 70 in das zylinderförmige Deckelteil 60 ermöglichen. Eine ebenfalls an der Innenseite des zylinderförmigen Deckelteils 60 vorgesehene Brücke 65 dient der Verschraubung der tellerförmigen Grundplatte 70 an dem zylinderförmigen Deckelteil 60.

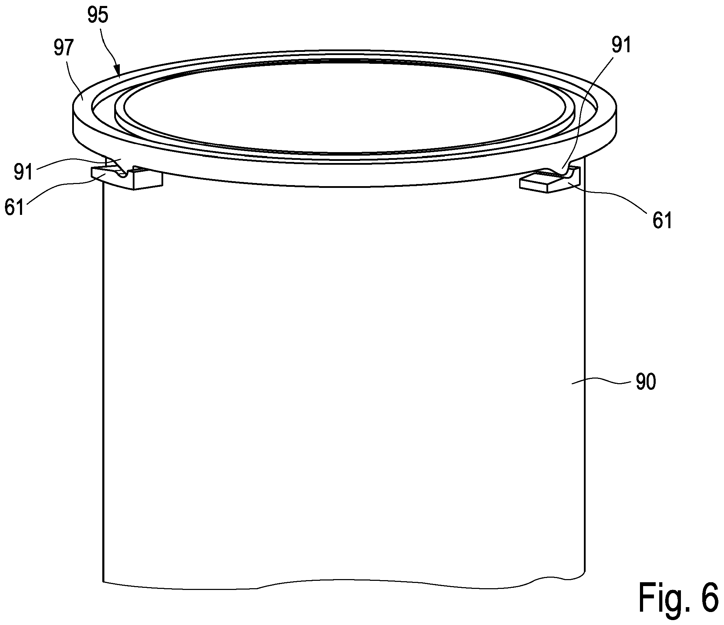

Um einen Druckwasserbehälter in dem zylinderförmigen Deckelteil 60 halten zu können, ist am inneren Umfang des zylinderförmigen Deckelteils 60 ein Rastelement 61 in Form einer Rampe angeordnet. Dies wird später mit Bezug auf Fig. 6 genauer erläutert.

Fig. 5 zeigt nunmehr das zylinderförmige Deckelteil 60 der Fig. 4 mit eingesetzter tellerförmiger Grundplatte 70. Die tellerförmige Grundplatte 70 weist eine Durchgangsöffnung 76 auf, über die die tellerförmige Grundplatte 70 mit dem Steg 65 (vgl. Fig. 4) des zylinderförmigen Deckelteils 60 verschraubt werden kann. Wie der Fig. 5 entnommen werden kann, ist die tellerförmige Grundplatte 70 vollständig innerhalb des zylinderförmigen Deckelteils 60 angeordnet. Gleichsam ist der hier nicht gezeigte Sterilluftfilter, der sich auf der Oberseite der tellerförmigen Grundplatte 70 befindet, vollständig innerhalb des zylinderförmigen Deckelteils 60 angeordnet. Eine korrespondierende Ausnehmung 73 an der tellerförmigen Grundplatte 70 dient in Zusammenwirkung mit der unterseitige Ausnehmung 63 (vgl. Fig. 4) dem drehlagekorrekten Einsetzen der tellerförmigen Grundplatte 70 in das zylinderförmige Deckelteil 60.

Mit Bezug auf Fig. 6 soll nun die Befestigung des Druckwasserbehälters 90 genauer erläutert werden. Aus Gründen der Darstellung ist das zylinderförmige Deckelteil 60 (vgl. Fig. 1 und 5) in Fig. 6 nicht dargestellt. Vielmehr sind in Fig. 6 als Teile des zylinderförmigen Deckelteils nur zwei Rastelemente 61 des Deckelteils angeordnet.

Insgesamt sind umfangsseitig drei Rastelemente 61 vorgesehen, von denen in Fig. 6 nur zwei ersichtlich sind. Die Rastelemente 61 des zylinderförmigen Deckelteils sind vorliegend rampenförmig ausgebildet. Der im vorliegend dargestellten Ausführungsbeispiel zylinderförmig ausgebildete Druckwasserbehälter 90 weist einen ringförmigen Kragen 97 auf, der zur Anlage an die tellerförmige Grundplatte 70 (vgl. Fig. 5) bestimmt ist. An dem Kragen 97 sind Rastelemente 91 in Form von Rastnasen angeordnet, die zu den rampenförmig ausgebildeten Rastelementen 61 des zylinderförmigen Deckelteils korrespondieren.

Um den Druckwasserbehälter 90 an der Behälteraufnahme der Betriebswasserversorgungseinheit 100 anzuordnen, wird der Druckwasserbehälter 90 koaxial zum zylinderförmigen Deckelteil 60 gehalten und in das zylinderförmige Deckelteil 60 eingeschoben. Durch eine leichte Drehbewegung, vorliegend im Uhrzeigersinn, können die korrespondierenden Rastelemente 91 des Druckwasserbehälters 90 mit den Rastelementen 61 des zylinderförmigen Deckelteils in eine Rastverbindung gebracht werden. Im vorliegend dargestellten Ausführungsbeispiel ist eine Drehung um weniger als 20° erforderlich, um den Druckwasserbehälter 90 im zylinderförmigen Deckelteil 60 einzurasten. Vorteilhafterweise kann somit ein besonders schnelles Wechseln des Druckwasserbehälters 90 erfolgen.

Um eine Abdichtung zwischen Druckwasserbehälter 90 und der tellerförmigen Druckplatte 70 zu gewährleisten, ist am Kragen 97 des Druckwasserbehälters 90 eine umlaufende Nut 95 ausgebildet, in die eine umlaufende Dichtung 75 (vgl. Fig. 3) eingreifen kann. Die umlaufende Dichtung 75 ist nicht notwendigerweise an der tellerförmigen Grundplatte 70 angeordnet. Die umlaufende Dichtung 75 kann vielmehr auch als separater Dichtring bereitgestellt sein.

Fig. 7 zeigt eine erfindungsgemäße Betriebswasserversorgungseinheit 100, die mit einer zahnmedizinischen Behandlungseinheit 200 und einer Druckluftquelle 300 verbunden ist. Eine Druckluftquelle 300 in Form eines Kompressors ist über eine Druckluftzuleitung 310 mit dem Drucklufteingang 10 der Betriebswasserversorgungseinheit 100 verbunden, so dass von der Druckluftquelle 300 stammende Druckluft DL in den Drucklufteingang 10 strömen kann.

Durch die eingeleitete Druckluft DL wird im Druckwasserbehälter 90 befindliches Betriebswasser BW über eine Betriebswasserzuleitung 230 der medizinischen Behandlungseinheit 200, vorliegend einer Dentaleinheit, zugeführt. In der als Dentaleinheit bereitgestellten medizinischen Behandlungseinheit 200 wird das Betriebswasser BW wiederum einem Bohrer oder einem Spülkopf zugeführt. Im in Fig. 7 dargestellten Ausführungsbeispiel ist der erfindungsgemäß vorgesehene Sterilluftfilter 50 innerhalb eines Gehäuses der Betriebswasserversorgungseinheit 100 angeordnet.

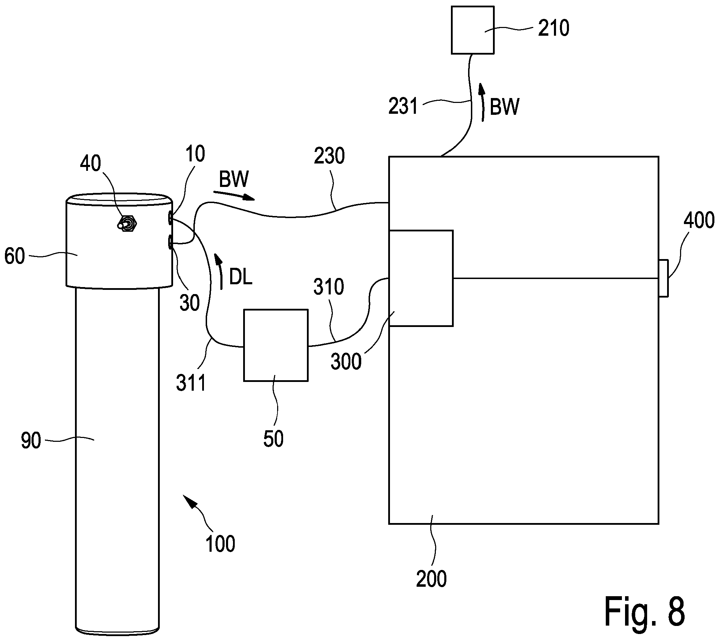

Fig. 8 zeigt eine mit einer medizinischen Behandlungseinheit 200 gekoppelte Betriebswasserversorgungseinheit 100. Im Gegensatz zu dem mit Bezug auf Fig. 7 beschriebenen Ausführungsbeispiel ist der erfindungsgemäß vorgesehene Sterilluftfilter 50 in einem Druckluftpfad zwischen einer zu der Betriebswasserversorgungseinheit 100 externen Druckluftquelle 300 und dem Drucklufteingang 10 angeordnet.

Der Sterilluftfilter 50 und die Druckluftquelle 300 sind über eine Druckluftzuleitung 310, die vorliegend kürzer als 2 Meter ist, miteinander druckluftverbunden. Über eine Filterleitung 311, die vorliegend kürzer als 2 Meter ist, ist Sterilluftfilter 50 ausgangsseitig mit dem Drucklufteingang 10 verbunden. Die Druckluftquelle 300, vorliegend ein Druckluftanschluss der medizinischen Behandlungseinheit 200 ist ihrerseits durch einen zentralen Druckluftkompressor 400 druckversorgt.

Über die Druckluftquelle 300 wird eine im Wesentlichen ungefilterte Druckluft dem Sterilluftfilter 50 zugeführt, von wo diese zum Drucklufteingang 10 gelangt. Wie bereits vorbeschrieben, gelangt das Betriebswasser BW aus dem Druckwasserbehälter 90 über die Betriebswasserleitung 230 zur zahnmedizinischen Behandlungseinheit 200. Verbunden mit der zahnmedizinischen Behandlungseinheit 200 ist ein Spülkopf 210, in den das Betriebswasser eingeleitet wird.

Alternativ zu den in Figuren 7 und 8 gezeigten Ausführungsbeispielen, bei denen die Betriebswasserversorgungseinheit 100 außerhalb der medizinischen Behandlungseinheit 200 angeordnet ist, kann die Betriebswasserversorgungseinheit 100 selbstverständlich auch innerhalb eines Gehäuses einer Behandlungseinheit 200 angeordnet sein.

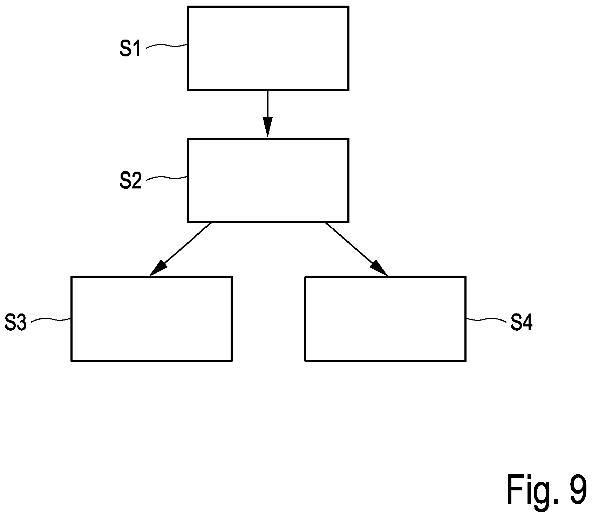

Fig. 9 zeigt beispielhaft ein erfindungsgemäßes Verfahren zum Betreiben einer Betriebsversorgungseinheit.

In einem ersten Schritt S1 wird eine insbesondere ungefilterte Druckluft von der Druckluftquelle zum Sterilluftfilter der Betriebswasserversorgungseinheit geführt.

In einem anschließenden Schritt S2 erfolgt ein Einleiten der durch den Sterilluftfilter der Betriebswasserversorgungseinheit gefilterten Druckluft in einen Druckwasserbehälter.

In einem Schritt S3, der typischerweise im Rahmen eines Behandlungsbetriebs erfolgt, ist der Druckwasserbehälter mit einem bevorzugt biozidhaltigem Betriebswasser gefüllt, so dass das Betriebswasser über einen Betriebswasseranschluss der Betriebswasserversorgungseinrichtung abgegeben wird, beispielsweise an einen Spülkopf oder Bohrer einer zahnmedizinischen Behandlungseinheit.

In einem alternativen oder zusätzlichen Schritt S4 ist der Druckwasserbehälter während einer Reinigungsphase im Wesentlichen frei von Betriebswasser, so dass ein dem Sterilluftfilter bzw. dessen Betriebswasseranschluss nachgeschalteter Betriebswasserpfad mittels der vom Sterilluftfilter gefilterten Druckluft freigeblasen wird.

### Bezugszeichenliste

- 10: Drucklufteingang
- 20: Druckluftanschluss
- 30: Betriebswasseranschluss
- 40: Schalter
- 50: Sterilluftfilter
- 60: zylinderförmiges Deckelteil
- 61: Rastelement am Deckelteil
- 62: Steg
- 63: Ausnehmung
- 65: Brücke
- 70: tellerförmige Grundplatte
- 73: Ausnehmung an der Grundplatte
- 75: umlaufende Dichtung
- 76: Durchgangsöffnung
- 80: Behälteraufnahme
- 90: Druckwasserbehälter
- 91: Rastelement am Druckwasserbehälter
- 95: umlaufende Nut
- 97: Kragen am Druckwasserbehälter
- 100: Betriebswasserversorgungseinheit
- 200: medizinische Behandlungseinheit
- 230: Betriebswasserzuleitung
- 300: Druckluftquelle
- 310: Druckluftzuleitung
- 320: Filterleitung
- 400: zentraler Druckluftkompressor
- BW: Betriebswasser
- DL: Druckluft

## Patentansprüche

1. Betriebswasserversorgungseinheit (100) zum Versorgen einer medizinischen Behandlungseinheit (200) mit Betriebswasser (BW), umfassend:
einen Druckwasserbehälter (90) zur Bevorratung von Betriebswasser (BW);
eine Behälteraufnahme (80), die zur Aufnahme des Druckwasserbehälters (90) ausgebildet ist; und
ein zylinderförmiges Deckelteil (60), das die Behälteraufnahme (80) einhaust;
wobei die Behälteraufnahme (80) umfasst:
einen Drucklufteingang (10), der zum Anschluss der Betriebswasserversorgungseinheit (100) an eine Druckluftquelle (300) ausgebildet ist;
einen Druckluftanschluss (20), der ausgebildet ist, die von dem Drucklufteingang (10) stammende Druckluft (DL) in den Druckwasserbehälter (90) einzuleiten;
einen Betriebswasseranschluss (30), der ausgebildet ist, das Betriebswasser (BW) durch die in den Druckwasserbehälter (90) eingeleitete Druckluft (DL) aus dem Druckwasserbehälter (90) herauszufördern und an die medizinische Behandlungseinheit (200) abzugeben;
einen Sterilluftfilter (50), der in einen Druckluftpfad zwischen dem Drucklufteingang (10) und dem Druckluftanschluss (20) eingebunden ist; und
eine tellerförmige, runde Grundplatte (70), durch die der Betriebswasseranschluss (30) und der Druckluftanschluss (20) zumindest abschnittsweise hindurch verlaufen.

2. Betriebswasserversorgungseinheit (100) nach Anspruch 1, ferner umfassend einen Schalter (40), der zur Unterbrechung/Freischaltung des Druckluftpfads zwischen dem Drucklufteingang (10) und dem Druckluftanschluss (20) ausgebildet ist.

3. Betriebswasserversorgungseinheit (100) nach Anspruch 2, wobei der Schalter (40) dem Sterilluftfilter (50) nachgeschaltet oder vorgeschaltet ist.

4. Betriebswasserversorgungseinheit (100) nach einem der Ansprüche 1 bis 3, wobei die tellerförmige Grundplatte (70) eine umlaufende Dichtung (75) aufweist, die den Druckwasserbehälter (90) zur Umgebung hin abdichtet, wenn dieser an der Behälteraufnahme (80) befestigt ist.

5. Betriebswasserversorgungseinheit (100) nach einem der Ansprüche 1 bis 4, wobei das zylinderförmige Deckelteil (60) an einem inneren Umgang wenigstens ein Rastelement (61) aufweist, das zum Einrasten eines korrespondierenden Rastelements (91) des Druckwasserbehälters (90) zum Halten des Druckwasserbehälters (90) an der Behälteraufnahme (80) ausgebildet ist.

6. Betriebswasserversorgungseinheit (100) nach einem der Ansprüche 1 bis 5, ferner umfassend einen Steril-Wasserfilter, der dem Betriebswasseranschluss (30) vorgeschaltet ist.

7. Betriebswasserversorgungseinheit (100) nach einem der Ansprüche 1 bis 6, wobei der Druckwasserbehälter (90) ein Volumen zwischen 100 ml und 5000 ml, bevorzugt zwischen 1000 ml und 1300 ml aufweist.

8. Zahnmedizinische Behandlungseinheit (200) mit einer Betriebswasserversorgungseinheit (100) nach einem der Ansprüche 1 bis 7.

9. HNO-Behandlungseinheit mit einer Betriebswasserversorgungseinheit (100) nach einem der Ansprüche 1 bis 7.

10. Verfahren zum Betreiben einer Betriebswasserversorgungseinheit (100) nach einem der Ansprüche 1 bis 7, aufweisend die Schritte:
- Führen von Druckluft (DL) von einer Druckluftquelle zu dem Sterilluftfilter (50) der Betriebswasserversorgungseinheit (100);
- Einleiten der durch den Sterilluftfilter (50) gefilterten Druckluft in den Druckwasserbehälter (90);
- wobei der Druckwasserbehälter (90) während eines Behandlungsbetriebs mit einem Betriebswasser (BW) gefüllt ist, so dass das Betriebswasser (BW) über den Betriebswasseranschluss (30) abgegeben wird; und/oder
- wobei der Druckwasserbehälter (90) während einer Reinigungsphase im Wesentlichen frei von Betriebswasser (BW) ist, so dass ein dem Sterilluftfilter (50) nachgeschalteter Betriebswasserpfad mittels der vom Sterilluftfilter (50) gefilterten Druckluft (DL) freigeblasen wird.

11. Verfahren nach Anspruch 10, wobei das Betriebswasser (BW) biozidhaltig ist.

## Claims

1. A process water supply unit (100) for supplying a medical treatment unit (200) with process water (*Betriebswasser-* BW), comprising:
a pressurized water container (90) for holding process water (BW);
a container receptacle (80) adapted to receive the pressurized water container (90); and
a cylindrical lid part (60) which encloses the container receptacle (80); wherein the container receptacle (80) comprises:
a pressurized air inlet (10) which is adapted to connect the process water supply unit (100) to a pressurized air source (300);
a pressurized air port (20) adapted to introduce the pressurized air (*Druckluft* - DL) stemming from the pressurized air inlet (10) into the pressurized water container (90);
a process water port (30) adapted to convey the process water (BW) out of the pressurized water container (90) by the pressurized air (DL) introduced into the pressurized water container (90) and to deliver it to the medical treatment unit (200);
a sterile air filter (50) integrated in a pressurized air path between the pressurized air inlet (10) and the pressurized air port (20); and
a plate-shaped, round base plate (70) through which the process water port (30) and the pressurized air port (20) at least partially pass.

2. The process water supply unit (100) according to Claim 1, further comprising a switch (40) adapted to disable/enable the pressurized air path between the pressurized air inlet (10) and the pressurized air port (20).

3. The process water supply unit (100) according to Claim 2, wherein the switch (40) is located downstream or upstream from the sterile air filter (50).

4. The process water supply unit (100) according to any one of Claims 1 to 3, wherein the disc-shaped base plate (70) has a circumferential seal (75) which seals the pressurized water container (90) from the environment when it is attached to the container receptacle (80).

5. The process water supply unit (100) according to any one of Claims 1 to 4,
wherein the cylindrical lid part (60) has, at an inner circumference, at least one snap element (61) adapted to snap into a corresponding snap element (91) of the pressurized water container (90) to retain the pressurized water container (90) at the container receptacle (80).

6. The process water supply unit (100) according to any one of Claims 1 to 5, further comprising a sterile water filter located upstream from the process water port (30).

7. The process water supply unit (100) according to any one of Claims 1 to 6,
wherein the pressurized water container (90) has a volume between 100 ml and 5000 ml, preferably between 1000 ml and 1300 ml.

8. A dental treatment unit (200) with a process water supply unit (100) according to any one of Claims 1 to 7.

9. An otorhinolaryngological treatment unit with a process water supply unit (100) according to any one of Claims 1 to 7.

10. A method for operating a process water supply unit (100) according to any one of Claims 1 to 7, comprising the steps of:
- guiding pressurized air (*Druckluft* - DL) from a pressurized air source to the sterile air filter (50) of the process water supply unit (100);
- introducing the pressurized air filtered through the sterile air filter (50) into the pressurized water container (90);
- wherein, during the treatment operation, the pressurized water container (90) is filled with a process water (*Betriebswasser -* BW) such that the process water (BW) is delivered via the process water port (30); and/or
- wherein, during a cleaning phase, the pressurized water container (90) is substantially free of process water (BW) such that a process water path downstream from the sterile air filter (50) is blown free by means of the pressurized air (DL) filtered by the sterile air filter (50).

11. The method according to Claim 10, wherein the process water (BW) contains biocides.

## Revendications

1. Unité d'alimentation en eau de service (100) pour l'alimentation d'une unité de traitement médicale (200) en eau de service (*Betriebswasser-*BW), comprenant :
un récipient d'eau pressurisée (90) pour le stockage d'eau de service (BW) ;
un logement de récipient (80), lequel est formé pour accueillir le récipient d'eau pressurisée (90) ; et
une partie de couvercle cylindrique (60), qui entoure le logement de récipient (80) ; le logement de récipient (80) comprenant :
une arrivée d'air comprimé (10), laquelle est formée pour le raccordement de l'unité d'alimentation en eau de service (100) à une source d'air comprimé (300) ;
une prise d'air comprimé (20), laquelle est formée pour introduire dans le récipient d'eau pressurisée (90) l'air comprimé (*Druckluft* - DL) provenant de l'arrivée d'air comprimé (10) ;
une prise d'eau de service (30), laquelle est formée pour évacuer hors du récipient d'eau pressurisée (90) et fournir à l'unité de traitement médical (200) l'eau de service (BW), par l'air comprimé (DL) introduit dans le récipient d'eau de service (90) ;
un filtre à air stérile (50), qui est intégré dans un chemin d'air comprimé entre l'arrivée d'air comprimé (10) et la prise d'air comprimé (20), et
une plaque de base (70) ronde en forme d'assiette que traversent la prise d'eau de service (30) et la prise d'air comprimé (20), au moins par sections.

2. Unité d'alimentation en eau de service (100) selon la revendication 1, comprenant en outre un interrupteur (40), lequel est formé pour l'interruption/l'activation du chemin d'air comprimé entre l'arrivée d'air comprimé (10) et la prise d'air comprimé (20).

3. Unité d'alimentation en eau de service (100) selon la revendication 2, l'interrupteur (40) étant disposé en aval ou en amont du filtre à air stérile (50).

4. Unité d'alimentation d'eau de service (100) selon l'une des revendications 1 à 3, la plaque de base (70) en forme d'assiette présentant un joint circonférentiel (75), lequel étanchéifie le récipient d'eau pressurisée (90) par rapport à l'environnement, lorsque celui-ci est fixé au logement de récipient (80).

5. Unité d'alimentation en eau de service (100) selon l'une des revendications 1 à 4, la partie de couvercle cylindrique (60) présentant au moins un élément d'enclenchement (61) sur un pourtour intérieur, lequel élément est formé pour l'enclenchement d'un élément d'enclenchement (91) correspondant du récipient d'eau pressurisée (90) pour le maintien du récipient d'eau pressurisée (90) dans le logement de récipient (80).

6. Unité d'alimentation en eau de service (100) selon l'une des revendications 1 à 5, comprenant en outre un filtre à eau stérile, lequel est disposé en amont de la prise d'eau de service (30).

7. Unité d'alimentation en eau de service (100) selon l'une des revendications 1 à 6, le récipient d'eau pressurisée (90) présentant un volume entre 100 ml et 5 000 ml, de préférence entre 1 000 ml et 1 300 ml.

8. Unité de traitement dentaire (200) avec une unité d'alimentation en eau de service (100) selon l'une des revendications 1 à 7.

9. Unité de traitement ORL avec une unité d'alimentation en eau de service (100) selon l'une des revendications 1 à 7.

10. Procédé pour l'exploitation d'une unité d'alimentation en eau de service (100) selon l'une des revendications 1 à 7, présentant les étapes :
- amener de l'air comprimé (DL) d'une source d'air comprimé au filtre à air stérile (50) de l'unité d'alimentation en eau de service (100) ;
- introduction dans le récipient d'eau pressurisée (90) de l'air comprimé filtré par le filtre à air stérile (50) ;
- pendant une opération de traitement, le récipient d'eau pressurisée (90) étant rempli avec de l'eau de service (BW) de telle sorte que l'eau de service (BW) est fournie par la prise d'eau de service (30) ; et/ou
- pendant une phase de nettoyage, le récipient d'eau pressurisée (90) étant essentiellement dépourvu d'eau de service (BW), de telle sorte qu'une voie d'eau de service disposée en aval du filtre à air stérile (50) est dégagée par soufflage au moyen d'air comprimé (DL) filtré par le filtre à air stérile (50).

11. Procédé selon la revendication 10, l'eau de service (BW) contenant un biocide.
